(19) **Europäisches Patentamt / European Patent Office / Office européen des brevets**

(11) **EP 1 839 279 B1**

(12) **EUROPEAN PATENT SPECIFICATION**

(45) Date of publication and mention
of the grant of the patent:
**22.06.2011 Bulletin 2011/25**

(21) Application number: **06716579.5**

(22) Date of filing: **18.01.2006**

(51) Int Cl.:
*G07D 7/20* (2006.01)    *G09F 3/00* (2006.01)
*C12Q 1/00* (2006.01)

(86) International application number:
**PCT/NL2006/000024**

(87) International publication number:
**WO 2006/078154 (27.07.2006 Gazette 2006/30)**

(54) **AUTHENTICATION METHOD AND SYSTEM FOR AUTHENTICATING SECURITY DOCUMENTS, SECURITY DOCUMENT AND SECURITY ELEMENT**

AUTHENTIFIZIERUNGSVERFAHREN UND SYSTEM ZUR AUTHENTIFIZIERUNG VON SICHERHEITSDOKUMENTEN, SICHERHEITSDOKUMENT UND SICHERHEITSELEMENT

PROCÉDÉ ET SYSTÈME D'AUTHENTIFICATION SERVANT À AUTHENTIFIER DES DOCUMENTS CONFIDENTIELS, DOCUMENT CONFIDENTIEL ET ÉLÉMENT DE SÉCURITÉ

(84) Designated Contracting States:
**AT BE BG CH CY CZ DE DK EE ES FI FR GB GR HU IE IS IT LI LT LU LV MC NL PL PT RO SE SI SK TR**

(30) Priority: **18.01.2005 NL 1028064**

(43) Date of publication of application:
**03.10.2007 Bulletin 2007/40**

(73) Proprietor: **VHP Veiligheidspapierfabriek Ugchelen B.V.**
**7339 GJ Apeldoorn (NL)**

(72) Inventor: **KRUL, Johannes**
**NL-6641 ZB Beuningen (NL)**

(74) Representative: **Wittop Koning, Tom Hugo**
**Exter Polak & Charlouis B.V. (EP&C)**
**P.O. Box 3241**
**2280 GE Rijswijk (NL)**

(56) References cited:
**WO-A-87/06383      DE-C1- 10 205 506**
**US-A- 4 442 214      US-A- 5 643 728**

**Description**

The present invention relates in general to the authentication of security documents.

According to a first aspect the invention concerns a method for authenticating a security document, wherein the security document comprises at least one of an enzyme and a substrate suitable for the enzyme, which method comprises the steps of

a) carrying out an enzymatic conversion between the enzyme and the substrate on the security document,

b) assessing a detectable change resulting from the enzymatic conversion where in the genetic information for the enzyme is derived from an extremophilic micro-organism.

According to a second aspect the invention relates to an authentication system for authenticating security documents, comprising at least an enzyme and a substrate suitable for the enzyme, so that the enzyme is capable of an enzymatic conversion in which a detectable resultant change occurs, and the security document comprises at least one of the enzyme and the substrate where in the genetic information for the enzyme is derived from extremophilic micro-organism.

WO 90/14441, EP 0327163, US 5 139 812, US 5 429 952, US 5 643 728 and US 5 942 444 are examples of authentication systems and methods in which security features are used which are taken from the living world.

DE 102 05 506 describes an authentication method for articles wherein a marker molecule can be bound to a binding molecule attached to a surface which is either on the article to be authenticated or on a second surface on a separate device. Recently, see WO 03/038000, it has been proposed that specific DNA and RNA single-strand fragments be used in such security features, compare WO 87/06383, in which only complementary parts or fragments hybridise on the bio-molecules. According to WO 03/038000 it is proposed that the complementary parts be provided with a "flag" in the form of a fluorescent substance at one end and a quenching group at the other end, so that the hybridisation bond between the bio-molecule and the complementary part thereof is easy to detect. The bound complementary parts show fluorescence, the non-bound parts do not. In this way it is possible to devise and produce almost infinite variants in which it is not easy for forgers to find out which precise polynucleotide sequence is significant.

Among the sources from the living world for developing security features, in practice little use has been made hitherto of the possibilities offered by enzymes. A possible reason for this is the lack of stability of many enzymes during normal use of an object protected by a security feature based on enzymes, either in the test conditions for determining the authenticity of the object by means of other tests, or in testing for other properties.

In the case for example of the manufacture of bank notes, belonging to the class of security documents which are usually protected with various marks of authenticity, allowance is made for the fact that bank notes are regularly left by accident in clothing which is then washed; the security features will have to be resistant to such washing treatment. Such washing will affect the stability of many enzymes. In such a case one speaks of denaturation of the enzyme. The original activity or function associated with the totality of the structure and/or composition of the bio-molecule in question is then lost. This activity or function, in particular the catalytic activity of bio-molecules, is very closely related to the three-dimensional structure, which is often fragile, i.e. this structure may change very quickly and often irreversibly as a result of disturbing influences. Increases in temperature are frequent causes of denaturation; sometimes even a decrease in temperature causes inactivation

Yet another disadvantage of the use of enzymes in security features is the relatively complex method that is needed for detecting the presence of the enzymes, and in particular the slow reaction rates.

It is an object of the present invention to provide authentication of security documents in which said disadvantages do not occur or at least occur to a lesser degree or provide a suitable alternative authentication.

This object is achieved according to the invention by virtue of the fact that in the authentication method and system the genetic information for the enzyme is derived from an extremophilic micro-organism. Extremophilic micro-organisms are micro-organisms that thrive in or require extreme conditions, such as high temperature (thermophiles), high pressure (barophiles; barotolerant bacteria) or a highly abnormal pH: (acidophiles pH $\leq$ 3; alkaliphiles pH $\geq$ 9) or combinations (polyextremophiles). The optimum degree of acidity for most non-extremophilic organisms is around the neutral pH of 7. The application of highly resistant enzymes in the authentication system according to the invention reduces the risk that as a result of normal use of the object to be protected the activity and function of the enzyme in question will be affected, and thus authentication will no longer be readily possible. Even under conditions which lie outside normal use this risk is still limited in the case of the invention. Furthermore, the use of enzymes from thermophilic organisms allows the authentication method, with which the authenticity of an object is checked, to be carried out at increased temperature, so that the enzymatic conversion takes place in a much shorter time. Just as in chemical reactions a temperature increase of about 10°C results in approximately a doubling of the reaction rate of the enzymatic conversion.

In this application "security document" means a document which is usually protected against fraud by at least one security feature. Examples include value documents, such as bank notes, deeds, tickets, vouchers, fiscal stamps, agreements and the like, identity documents such as passports, identity cards, visas, bank cards, credit cards, access documents, entrance tickets and labels such as authentication brand labels, tamper evidence labels and seals. Also packaging material, in particular for pharmaceutical, cosmetics, electronics or food industry may comprise one or more security features in order to warrant the content of the packaging like for instance genuine drugs. The paper of which such documents are produced is called "security paper". Such paper is produced in a secured manufacturing environment. This paper may comprise already all security features to be present later in the document made thereof. However additional security features may be added to the paper by e.g printers etc. to create the full security document. A document will always comprise data/text whereas security paper will lack this kind of information most of the time. In the context of this application ""security paper" is comprised in the expression "security document". I.e. the authentication method and system according to the invention are applicable to security documents, as well as to "blank" security paper.

Thousands of enzymes are known, and even many more substrates that can be converted by enzymes; both the enzymes as well as the substrates can be fully natural or modified. For the enzymes, by "natural' is meant that the substance at hand is produced by a not consciously modified biological species and isolated and purified thereafter, whereas by 'modified' is meant either a substance that is produced by a biological species which substance underwent a chemical modification after isolation and/or purification or a product produced by a biological species that is coded by consciously modified genetic information in/by that species. For substrates more or less the same holds; however a substrate may also be fully synthetic. The advantage of reactions catalysed by enzymes, relative to ordinary chemical conversions, is the often high specificity that enzymes show for the substrate to be converted, and in addition the fact that the conversions take place under relatively mild conditions, whereas often much more severe/harsh conditions are required for the analogous chemical conversions. Furthermore, the chemical conversions are usually not very (stereo) specific, whereas the enzymatic activity is linked precisely to the three-dimensional structure and size of the substrate (stereo-, region- and chemoselectivity and specifity).

Most bio-molecules with a catalytic activity are known to be proteins but certain RNA molecules also show a catalytic activity. It is also known that catalytic antibodies can be raised for certain substrates. These antibodies are raised against chemical structures which resemble the transition state of the substrate during an enzymatic conversion (the activated form of the substrate). In the present description the term "enzyme" will mean natural and artificially modified bio-molecules that exhibit a catalytic activity which is, or is not, stereospecific.

In the authentication method and system according to the invention the security document contains at least a substance chosen from the enzyme and the substrate. In performing authentication with the aid of the invention one can proceed in various ways. Usually, for testing the authenticity the other substance which is not present in or on the security document is brought into contact with the substance which is present in or on the security document, in order to initiate the enzymatic conversion. If desired, other materials to be involved in the enzymatic conversion may already have been provided in or on the security document, or may be added at the time of the test, or a combination of both. Examples of such materials are discussed below. The security document contains at least one of the substances chosen from enzyme and substrate. The security document may also contain both the enzyme and the substrate, provided that the enzymatic conversion cannot start spontaneously. A suitable example is a security document where the enzyme and the substrate are provided in separate regions. Another example involves physical separation of the enzyme and the substrate like for instance in microcapsules. If additional substances such as co-enzymes and/or co-factors are required for the enzymatic conversion, these may or may not be present in or on the document substrate, or may be added later during actual authentication.

In a preferred embodiment of the method and system according to the invention, the genetic information for the enzyme is derived from a hyperthermophilic or thermophilic micro-organism. Such enzymes have very high resistance against increased temperatures, as is apparent for example from the occurrence of bacteria in thermal springs. The advantage of using enzymes from (hyper)thermophilic organisms in vitro is that: the catalytic activity persists for a long time at increased temperature, the conversion rate at increased temperature is high in comparison to that at room temperature, and the denaturation does not occur quickly at the normal temperature of use.

It is known that enzymes from (hyper)thermophiles are used in the multiplication of (fragments of) DNA molecules (PCR-technique). In so far as the genetic information is concerned, the DNA polymerase needed for this purpose is derived from a micro-organism which occurs in thermal springs. When during the PRC reaction double-strand DNA is melted (the double strand unwinds and single strands become available for binding of the primers and further reproduction) at increased temperature, this polymerase enzyme retains its catalytic activity, so that the cycle of multiplication can restart each time without the need to add fresh enzyme.

In order to obtain large quantities of enzyme from specific organisms in a convenient manner, the information coding for the enzyme is often introduced into a micro-organism which is easy to manipulate and culture. Genetic manipulation also ensures that this organism produces a relatively large amount of enzyme (a so-called "overproducer"). The enzyme is isolated and, depending on the desired use, purified to a lesser or greater extent. The genetic information of an extremophilic micro-organism might also be incorporated into another living organism, passing thereby the species barrier, such that this other organism starts to produce the enzyme according to the obtained genetic information.

The enzymatic conversion rate is a function of many variables, such as temperature, enzyme concentration, substrate concentration, co-enzyme concentration, the presence or absence of co-factors, activators, inhibitors and the like. A co-factor is a non-protein compound which is involved in the catalytic reaction but ultimately re-emerges unchanged from the catalytic process in question. A co-enzyme is a non-protein compound directly participating in the catalytic reaction, which e.g. can be regenerated via other enzymatic reactions. The distinction between co-enzyme and co-factor is not always that sharp.

There are many types of inhibitors varying from types which very specifically affect only the catalytic action of certain enzymes to types which affect the integrity of proteins or the structures thereof in general. In addition, one can also classify inhibitors according to the type of binding, namely the reversibly and the irreversibly binding ones. Very strong binding of an inhibitor to an enzyme, or a covalent modification of an amino acid which is in some way crucial for the catalysis and/or structure of the catalytic site, leads to irreversible inhibition (strong binding here means: strong relative to binding of the substrate and/or another factor needed for the enzymatic conversion) .

A recent class of inhibitors, applicable in the specification is nucleic acid ligands. Such nucleic acid ligands are able to complex with the most diverse compounds, including proteins. See for example US-A-6083696, in which ligands are described with which an inhibition of elastase by an inhibitor peptide increases 30,000 times when that inhibitor peptide was bound to a specific nucleic acid ligand. The effect on the enzyme action was found to be very specific, since other serine proteases were scarcely influenced by this inhibitor combination. Combination of an inhibitor with a nucleotide which weakens the inhibition is also conceivable.

Other inhibitors that can be used in the specification are those which nullify the catalytic action of enzymes less specifically because they very generally affect the integrity of a protein (structure) and/or can bind to the side chains of a particular amino acid. The effect of modification is far-reaching, especially if the amino acid residue modified in this way plays an essential role in the structural integrity of the bio-molecule and/or in the catalytic process. The effects of heavy metals (*inter* alia binding of sulphur groups) and of the inhibitors of nervous function, such as the specific binders of -OH groups of serine, are well known.

There are enzymes of which the kinetics cannot be described with the Michaelis-Menten model (see below). The binding of the substrates to these enzymes exhibits a much more complex ( cooperative) behaviour; as a consequence the kinetics for such enzymes deviate from the Michaelis-Menten kinetics. Such enzymes are called 'allosteric' and they are often found in biochemical pathways at regulatory positions. At a constant substrate concentration this type of enzyme may catalyse at a different rate under the influence of the so-called allosteric effectors. In this description these and other effectors are simply called inhibitors or activators, according to their effect on the enzymatic conversion rate at a particular substrate concentration.

The enzyme is advantageously stabilized, so that it is even better protected against the conditions of use of the authentication system than it already is *per se*. Examples thereof include adhesion to a solid support or carrier, or confinement therein, e.g. in a particulate support. Stabilization by a bio-molecule which does not take part in the reaction, such as a protein and/or poly-nucleotide, is a further possibility. It is also possible to further protect an enzyme by the immobilisation/incorporation in a water retaining material such as a polyurethane. See e.g. Koepsel and Russel (Science, 309, p 377; www.sciencemag.org) For the purpose of the invention the enzyme and eventually a (limited) number of other compounds required for the reaction can be used in a coating covering fully or partially a security paper or security document. A fully coated article allows for a simple and quick authentication. A locally coated article presents a higher level of protection. In a multilayered paper the enzyme and/or substrate may also be present in an intermediate layer, or in different layers if both components are present.

In a preferred embodiment of the invention the enzyme is present in a starting reagent, which starting reagent comprises an enzyme stabilizing substance or means. The starting reagent is here defined as the last addition which takes place in order to initiate the enzymatic conversion. This substance is preferably chosen from the group which includes a co-enzyme, a co-factor or a substrate or one of the substrates in the case of multi-substrate reactions. The starting reagent or the security document can also advantageously include an enzyme activator and/or an enzyme inhibitor, so that the enzymatic reaction can be controlled and/or modified in a predetermined way.

The enzymatic conversion can belong both to the class of single-substrate reactions as well as to the class of multi-substrate reactions.

By using an enzyme stemming from the genetic information of an extremophilic enzyme the chance of a genuine article (i.e. not counterfeit) being wrongly regarded as false in the authentication is small. This increases the reliability of the method for authenticating security documents. The security document is advantageously provided with the enzyme. With a view to the rate of the authentication method the enzymatic conversion is advantageously performed at elevated temperature, advantageously in a range between 50 and 95°C. Compared with the enzymatic reaction taking place at room temperature, the speed in this temperature range is generally many times higher (4-100 to 200 times), as a result of which the time required for the test will be considerably reduced.

The enzymatic conversion is advantageously linked to a chemical and/or enzymatic follow-up reaction. Although this makes the authentication *per* se more complex, this follow-up reaction increases the level of protection of the security document. Preferably a very low concentration of a substrate, or another compound involved in the conversion, is present in/on the document the presence of which compound is proven by recycling the reaction product of it in the follow-up reaction; this allows to employ only trace amounts of a certain substance the presence of which nevertheless can be proven in this way.

The reaction conditions for performing the enzymatic conversion, such as pH, ion strength, etc., are advantageously kept constant over at least that part of the surface of the security document to be tested.

When a higher level of protection is required, one may also vary the reaction conditions during the performance of the method, depending on the nature of the reagents on the objects to be protected. If, for example, during testing the enzyme is present or applied in discrete regions which lie at a certain distance from each other, it is possible to have a first enzymatic reaction taking place at room temperature, and then to change (increase) the temperature at each transition to a further region, or vice versa. The reaction constants or rates, which can thus be measured, give a unique "fingerprint" of the document in question. In an alternative embodiment it may also be that in one area of the security document, in different preferably not overlapping parts thereof, the test is performed, e.g. using a line printing device.

One can carry out enzymatic reactions with the same enzyme with several substrates, preferably in discrete regions, so that even more specific information on the authenticity can be obtained.

A double check using different enzymes at least one of which is derived from an extremophilic micro-organism is also within the scope of the invention.

Step b) of the method according to the invention can include the visual assessment of the resultant change, a change in colour for example. The resultant change can also be read by machines like (spectro)photometers, in which case, if desired, the degree and/or rate of change can be a further indication of the authenticity. The assessment step may also include the investigation of an olfactory change or of a tactile change.

In a preferred embodiment the enzymatic conversion rate is determined, as will be explained at length below.

According to a third aspect the invention concerns a security document as defined hereinbefore including value documents, such as bank notes, deeds, agreements and the like, identity documents such as passports, identity cards, visas, bank cards and credit cards, vouchers, stamps, access documents, (entrance) tickets, seals and labels; also packaging material as described earlier may comprise security features in order to warrant the content of the packaging like for instance genuine drugs (such packaging material is contained in the expression "security document") which document has been provided with a security feature based on an enzymatic conversion between an enzyme and a substrate. According to the invention the security feature comprises an enzyme the genetic information of which is derived from an extremophilic micro-organism. Preferably the security documents used in the invention comprise at least one common security feature and an enzymatic conversion based security feature. Examples of such common security features are security threads/foils, watermarks, optically variable devices like colour switching ones, interferential structures and holograms, fibers (dichroic, fluorescent, magnetic), luminescent features with emissions in and outside the visible region, magnetic features, conductance, specific chemicals and microprint etc.

The preferred embodiments discussed above are also applicable to the security document according to the invention.

In a preferred embodiment of the invention the enzyme is present or applied over at least part of the surface of the security document in one concentration. This embodiment will generally be used when a rapid and relatively simple authentication is desired. This is the case, for example, when one uses only the presence or absence of a specific enzyme reaction for the determination of authenticity.

It will be clear that in the method and system according to the invention at least one of the required reagents (enzyme and substrate) is present in or on the security document to be tested, and the other one is brought into contact therewith during testing.

In an alternative embodiment the enzyme is present or applied in/on discrete regions of the surface of the security document. The security document and element as claimed comprised the enzyme. This allows the concentration of the enzyme or other required reagents to be varied over the various regions, so that specific characteristics of the enzymatic conversion that are related thereto can be determined, such as, for example, concentrations, degree of change, conversion rate, reaction constants, etc., which can vary from region to region. Such a verification or authentication is complex, so that it will generally only be applied in the case of very valuable and unique documents. In order to increase the level of security further, a protein such as a second enzyme can be present in the authentication system/security document as a masking background. The concentration of this protein may for example be much greater than that of the first enzyme, while the first enzyme is the enzyme which causes the desired conversion in the determination of authenticity. If necessary one can also add non-catalytic proteins like bovalbumin or ovalbumin.

In yet another embodiment the security document according to the invention comprises at least two different enzymes, which are provided in two regions that at least partially overlap each other.

In the invention one can make use of a number of very characteristic kinetic properties of enzyme systems under all kinds of variable conditions. These properties cannot be falsified except by a completely identical feature, which, in view of the number of possible variants, is almost impossible to copy via "reverse engineering".

The use of enzymes for analytical purposes normally is the determination of a substrate concentration/quantity in a sample. Two different approaches can be generally distinguished, namely endpoint determination and the kinetic approach.

One speaks of an endpoint determination when all the substrate to be determined is completely converted, so that the difference between the initial state and the final state in the test constitutes an unambiguous quantitative measure of the concentration(s)/quantities to be determined. In order to achieve a complete conversion in some cases one applies auxiliary steps, such as causing follow-up reactions to take place through which always a possibly unfavourable establishment of equilibrium in the first reaction is counteracted and/or measurable signal changes occur. By the use of for instance a large excess of a second substrate (water, for example, if needed as a second substrate) one also imposes a shift in the equilibrium concentration(s) such that the first substrate (the one to be measured) is converted almost quantitatively. Such a difference between the initial and final state constitutes a quantitative measure of the concentration(s) to be determined. The quantities/concentrations of enzyme to be used largely determine the speed at which the final state is established, keeping the rest of the reaction conditions constant. The precise quantities of enzyme used are not critical in an endpoint determination as long as the substrate concentration to be determined is many times greater than the enzyme concentration; or in other words: the quantity of substrate which is bound to the enzyme must be negligibly small relative to the quantity not bound to the enzyme.

In the endpoint determination the temperature is not very important, provided that one can be sure that the substrate conversion will be complete in the interval of time available for the determination. As a raised temperature increases the rate of the enzymatic conversion the endpoint is reached earlier; of course the equilibrium concentrations are depending on the temperature, but under the rest of the reaction conditions for the endpoint determination the temperature shift of the equilibrium will not have a significant effect on the outcome of the substrate concentration.

If it is desirable to work faster in determining an unknown substrate concentration, a kinetic determination may be performed. This allows an accurate determination of an unknown substrate concentration to be performed in just a few seconds on the basis of an enzymatic conversion rate, provided that the calibration data for the rates of at least one known concentration of the same substrate under identical test conditions are known.

The enzyme rate of a single-substrate reaction in the steady state is given in the Michaelis-Menten equation. If a second substrate is present in very great excess relative to the first substrate the reaction can also be considered to be a single-substrate reaction (pseudo single-substrate reaction); water, for example, is such a second reactant, present in great excess (about 55,5 M), in the conversion of p-nitrophenylphosphate catalysed by alkaline phosphatase under the usual test conditions.

In the derivation of the rate equation for a single-substrate reaction, shown in reaction equation 1 below, it is assumed that the substrate concentration ([S]) will be many times greater than that of the enzyme ($[E]_0$) and that the quantity of substrate is initially many times greater than that of the product ([P]). These assumptions usually also apply to in vitro enzymatic tests. It is also assumed that in the first reaction (see below) an equilibrium is established while, initially, in the second reaction no equilibrium has yet been established ([P] very low). It is also assumed that: d[ES]/dt =0; called the "steady state" assumption.

$$\text{Reaction equation 1: } E + S \Leftrightarrow ES \quad E + P$$

The reaction rate at the beginning of the reaction is then given by

$$V = \frac{k_{cat}[E]_0[S]}{K_M + [S]}$$

in which:

v is the rate initially measured;
$[E]_0$ is the total enzyme concentration;
[E] is the free enzyme concentration, at very low substrate concentration $[E] \approx [E]_0$;
[S] is the free substrate concentration (but because $[E]_0 \ll [S]_0$, under ordinary test conditions $[S] \approx [S]_0$);
$[S_0]$ is the total substrate concentration;
$k_{cat}$ is a catalytic constant characteristic of the enzyme under the given test conditions;
$K_M$ is an apparent dissociation constant which can serve as an overall dissociation constant for all enzyme-bound forms under the given test conditions (generally: $K_M = [E] [S]/ \Sigma$ [ES], in which the nominator is the sum of all bound enzyme forms); the product $k_{cat} [E]_0$ is called $V_{max}$.

Under the condition that $[S] >> K_M$, the rate (v) of the measured reaction is almost equal to that of $V_{max}$, and thus directly proportional to the quantity of enzyme used and not proportional to that of the substrate concentration; under such conditions the enzyme is saturated with substrate, and increasing the substrate concentration hardly leads to a higher conversion rate, or not at all.

When $[S] << K_M$, the reaction rate is directly proportional both to the enzyme and to the substrate concentration; because of the direct proportionality to $[S]$, this is the condition in which a kinetic substrate concentration determination will preferably be performed.

The rate equations for multi-substrate reactions are more complicated than the equations given above; moreover, the rate equations are also dependent on the mechanism of the enzyme in question. Conversely, from a complete rate equation one can work out which enzyme mechanism is involved; enzyme mechanism here means the sequence of the binding of the substrates and co-enzymes and also of the release of the conversion products during the entire catalytic cycle.

The $K_M$ of a particular enzyme for a particular substrate is a characteristic datum in an enzyme catalysed reaction under given conditions. The $K_M$ for the same substrate may be completely different for enzymes which catalyse the same reaction but which originate from a different organ (iso-enzymes) or different organism. Although $K_M$ is not a direct dissociation constant, $K_M$ is representative for the affinity of the enzyme for the substrate in question under the given conditions; interference with the enzyme can lead to a change in the $K_M$, especially if this interference affects the substrate binding site, e.g. through a change of charge in an amino acid side chain and/or through a conformation change in the protein and/or through steric obstruction/change (this last may result from a change in the size of amino acid side chains).

It is possible to determine the $K_M$ for a particular substrate by measuring at different substrate concentrations the corresponding initial rates and a known (usually constant) enzyme concentration/quantity; in that case the other concentrations, such as those of a possible second substrate and of co-enzymes and co-factors, are usually set at high (satuxation) levels.

In order to measure the $K_M$ it may suffice to do only one measurement with a known initial concentration of the substrate and to observe this reaction over time.

In both cases the $K_M$ of the substrate-enzyme system in question can be calculated and/or determined via all kinds of graphical methods and/or algorithms.

The $K_M$, as determined from a Lineweaver-Burk plot, for a particular substrate apparently becomes different in the presence of particular inhibitors; this is due to the fact that this apparent $K_M$ then also contains data with regard to the inhibition which data are characteristic for the inhibitor used in the given conditions.

By the same methods as mentioned above, one can also obtain the $V_{max}$ of an enzymatic system. This quantity is proportional both to the catalytic constant ($k_{cat}$) and to the amount of enzyme used. The $V_{max}$ apparently changes in the presence of particular inhibitors; from this change, at a given inhibitor concentration, it is possible to measure the inhibition constant ($K_I$) belonging to this kind of inhibitor. There are also inhibitors which apparently change both the $K_M$ term and the $V_{max}$ term in such a way that the ratio of the two remains constant. Finally there are inhibitors which give rise to a mixed inhibition pattern. In all cases of inhibition, however, one is able to determine the $K_M$, $V_{max}$ and $K_I$ provided that the other reaction conditions are known like substrate concentration, inhibitor concentration etc.

In the determination of a substrate concentration for the conversion at stake one generally chooses an enzyme that has a high conversion rate, together with good stability and specificity. The conversion rate at a given $[S]$ and $[E]$ is a function of the catalytic conversion constant $k_{cat}$ and the binding constant $K_M$. Generally speaking, a high $K_M$ combined with a high $k_{cat}$ is favourable for a rapid conversion. (See for example "Structure and mechanism in protein science", 3rd ed. 1999, Alan Fersht, W. H. Freeman and Company, New York, p. 362).

In the previously mentioned Michaelis-Menten enzyme rate equation one does not explicitly find the influences of all kinds of factors which are usually kept constant during the test like the pH and the temperature. Such factors however each have a marked influence on an enzymatic reaction rate.

The influence of temperature change is generally the same as that which would occur if the reactions were able to proceed uncatalysed. If the temperature becomes too high, however, the catalysed reaction will stop abruptly because of denaturation of the biocatalyst. The use of enzymes from extremophilic and, in particular, hyperthermophilic micro-organisms reduces the risk of denaturation by an elevated temperature.

Upon exposure to extreme pH values many bio-molecules will denature, and in the range where denaturation does not yet occur, the pH may have a great effect on the catalytic rate of an enzyme-catalysed reaction. In in vivo tests in which the conversion rates of enzymes are studied or in which enzymes are used for the determination of concentrations, the pH is usually kept constant by pH buffers. The chemical nature of the buffer itself can also influence the rate of the conversions, as can also the concentration thereof. This last is usually attributable to ionic strength effects on the binding of the enzyme and the substrate. Changing the salt ion concentrations also influences the ionic interactions in the enzyme such that there may be a change in the three-dimensional structure and, associated with this, the catalytic activity. If the concentration of salt becomes very high, there are salting-out effects which are due to competitive (de)hydration; a protein may then precipitate without this necessarily having to lead irreversibly to a loss of catalytic activity.

It is noted here that an enzyme helps to reach a faster establishment of the equilibrium. An enzyme is not able to change the position of an equilibrium. The equilibrium position, at a given temperature and pressure, is determined solely by the thermodynamic data of the reactants (substrates, co-enzymes, products) involved in the conversion, i.e those substances that are not regenerated in the catalytic cycle. It may happen, however, that in spite of the fact that the reaction is catalysed by an enzyme the equilibrium is not established.

For the determination, according to the invention, of the presence of a particular compound in the security document it is sufficient that a specific change occurs. This change may be directly perceptible by the normal human senses and/or machine readable. These sensorily perceptible changes may be visual and/or olfactory and/or tactile. Sometimes for a sensory perception one also needs a simple tool such as a magnifying glass or a so-called black light (uv light).

All possible ways of measuring enzyme activities/enzymatic conversions are in principle suitable for the invention.

For measurable/quantifiable changes one usually employs spectral techniques. These changes may manifest themselves as a change in absorption, reflection, fluorescence or phosphorescence, and all this in the UV and/or visible and/or near IR region. By far the most enzyme activities are measured (spectro)photometrically or fluorimetrically. Other more specifically applicable examples include inter alia bioluminescence, with which one is able for example rapidly to measure very low ATP concentrations. The (chemical) energy from the energy-rich compound ATP is transferred to a fluorescent group during the so-called luciferase reaction. The amount of light emitted is directly proportional to the amount of ATP present and thus also of the presence of, for example, an ATP producing organisms; the presence of microbial contaminations can be established in this way. Enzyme activities may also result in a change in optical activity, e.g. a change in the polarizing angle; one example is that of the effect of sucrase on sucrose, where the dextrorotation of a sucrose solution changes to a levorotation after the cleavage of the glycosidic bond between D-fructose and D-glucose; the specific levorotation of D-fructose is greater than that of the dextrorotatory D-glucose. Sometimes one measures an enzyme activity by titrating substrate groups released during the reaction.

A great number of conditions play a role in the stability of enzymes (in this case the catalytic, activity), including the thermal stability. As can be expected this thermal stability is high in the case of enzymes which are coded by genetic information of (hyper)thermophilic micro-organisms. Depending on the kind of security document, and the normal conditions of use thereof, it may also be significant whether enzyme inactivation, for example, can occur as a result of oxidation with oxygen from the air, or through sensitivity to particular substances, such as (traces of) heavy metals. The possibilities for direct incorporation of such a sensitive enzyme into an object to be authenticated can be limited as a result of this. On the other hand one can also make use of precisely these sensitivities of an enzyme. It is possible to use an enzyme that is oxygen sensitive but that is fully protected from the air by a coating and/or barrier layers; if one tries to tamper with the document the protecting layers might be disturbed and during testing no activity will be found from the inactivated enzyme. A tamper indication might also be achieved if at least one of the enzyme(s) is susceptible to oxidating- and/or reducing- and/or organic compounds/solutions that are used in an attempt to alter data in/on the document. Then no enzyme activity is found as a result of prior denaturation of the enzyme under the tampering conditions.

In still another example, the security document can be locally provided with an inhibitor in a particular pattern (e.g. in the form of a visible and/or measurable (bar) code or geometric figure), while the other reactants are homogeneously distributed. When testing for authenticity, by adding test reagent , it will become clear that the reaction is not/ hardly taking place at places with an inhibitor, whereas the reaction is taking place much faster in the immediately adjacent area. Such a system can also be applied, *mutatis mutandis,* with an activator (distribution) instead of an inhibitor.

In order to stabilize enzymes in vitro, a number of possibilities are available. Commercially available enzymes are stabilized in various ways. Sometimes they are supplied in dried form (as a lyophilizate), sometimes in a high concentration of ammonium sulphate, sometimes in a glycerol solution with or without a buffer, sometimes in a high concentration of simple salt. (NaCl), sometimes only in a buffer., sometimes EDTA (ethylenediaminetetraacetic acid) is added, and sometimes the enzymes are supplied with one of the substrates or with BSA (bovine serum albumin).

One can immobilize enzymes on or in (solid) carriers. Binding to a solid carrier can improve stability; the kinetic parameters may change. The incorporation of enzymes in solid carriers might also influence kinetic parameters by for instance diffusion limitations or by a change of polarity of the direct environment of the enzyme. Already the stabilisation in a coating has been mentioned earlier. A different way of stabilisation of at least one enzyme activity opens the way for a number of different ways of protecting a document. For instance an enzyme is present under different stabilising conditions and thus the activity of the enzyme will vary accordingly.

A specific way of binding proteins is known from US 6287765, where at least one protein (enzyme) is bound to polyfunctional polynucleotide. By bringing several proteins (enzymes) together in a specific/functional composition via this polyfunctional nucleotide polymers one is able *inter alia* to couple several enzymatic reactions in a concerted way. This last is, to a certain extent, comparable with multi-enzyme complexes, although in these last the whole three-dimensional/structural organization is optimally adjusted for the efficient running of several consecutive reactions; known examples of these last are the alpha-keto acid dehydrogenase complexes and the fatty acid synthetizing complex.

It is clear from the above that there are many different possibilities for stabilizing all kinds of enzymes; each kind of enzyme needs an individual approach. An optimum way of preserving/stabilizing is of great importance to assure the reproducibility of a quantitative test on the feature in the best possible way.

Most enzyme substrates are fairly to very stable under normal atmospheric conditions at room temperature. The stability of co-factors and co-enzymes varies from extremely stable to highly unstable. Co-factors in the form of metal ions (e.g. $Mg^{2+}$) are for example extremely stable but co-enzymes with a high energy content, such as. NAD(P)H or ATP, are not very stable at room temperature under atmospheric conditions.

For application of the invention to a security document it may be necessary for the reactants, enzymes and other requisites needed for the enzymatic conversion, to be protected against influences which might affect the document during normal use, e.g. water-soluble chemicals in documents subject to damp conditions. Examples of applicable protections include the storage of chemicals in microcapsules, with these chemicals not being released until the starting reagent is added, either by dissolving the capsules or by a mechanical effect. It is also possible to bind a naturally water-soluble reagent via a spacer to a solid matrix, e.g. to some part of a feature and/or the document. Finally, it is possible to immobilize water-soluble substances by attaching them to water insoluble micro particles and if needed via spacers. If on the other hand the substrates in a feature are of a strongly non-polar nature, there is much less/no danger of a washing out effect in a polar environment, and such measures do not *per se* need to be taken.

An enzyme test is generally performed in such a way that an initial state is measured in which no enzymatic conversion has yet occurred; then a last addition is made as a result of which the reaction starts and progresses. A last addition will thus always have to include at least one or more reagents which are essential for starting the reaction; this may be an enzyme and/or a missing substrate and/or a missing co-enzyme (and possibly a missing co-factor). The last addition may also include a mixture of several factors, such as an enzyme and one of the substrates and/or a co-enzyme. It is also possible that in the last addition the enzyme is missing because the latter, in one example of the feature, is already present in/on the substrate of the feature to be tested. Another possibility includes that at least part of the substrates for one enzymatic reaction is present in the starting reagent, the corresponding enzyme being present in the document, as well as another enzyme for which at least part of the substrates is present in/on the document. Care must be taken to make sure that, prior to the test, a spontaneous conversion cannot have occurred already either in the last addition or in/on the document to be tested. It is also conceivable to add in the last addition something through which a follow-up reaction starts; such a first unfavourable establishment of equilibrium may be entirely shifted to that of an "irreversible" reaction.

A minimum embodiment of the authentication system according to the invention includes all the requisites for at least a qualitatively and/or quantitatively reproducible enzymatic conversion. In addition one can also vary the conditions which can influence at least one enzymatic conversion.

It is possible that many substances are present which are entirely inert in relation to at least one enzymatic conversion and which thus do not influence this conversion, but which do have a characteristic of their own such as, colour, luminescent properties, conductivity, optical activity like polarising effects and magnetic properties, etc. One can also include all kinds of substances which simply serve to make it difficult for a forger to find out which substances are actually important in the system. Thus, for example, one can use a mixture of (stereo- )isomers, in which only one of these isomers is important, and in addition, if necessary, also use masking compounds which are not active in the authenticity test. One might also include a number of irrelevant (inert) nucleotide sequences in the case of a desired (enhanced) inhibition by a poly-nucleotide sequence.

In another variant the security document contains at least locally different substrate concentrations, where, after addition of the starting reagent, conversions take place at an equal initial rate because, under the conditions, the reaction does at the outset proceed under $V_{max}$ conditions. However the final result of the conversions is different as the product concentrations will be different. In another variant an enzymatic reaction may take place, at locally different substrate concentrations, and at the same time the different conversion rates are measured. The relevant kinetic parameters for the system can be established/calculated from these measurements.

According to yet another variant, discrete regions are designed in such a way that in one region an uninhibited reaction is in progress while in/on at least one other part of the mark the same enzymatic conversion is taking .place under inhibited conditions. In other variants one can work with activators, or with combinations of activators and inhibitors. The present invention is further explained below with the aid of the appended drawing, in which:

Fig. 1 is an embodiment of a security document with a local security feature;
Fig. 2 is a security feature with several component surfaces;
Figs. 3-6 show security features with a few variations per component surface;

Figs. 7-11 show security features in the form of a bar-code with a few variations per bar;

Fig. 12 is a feature which is made up of various signs.

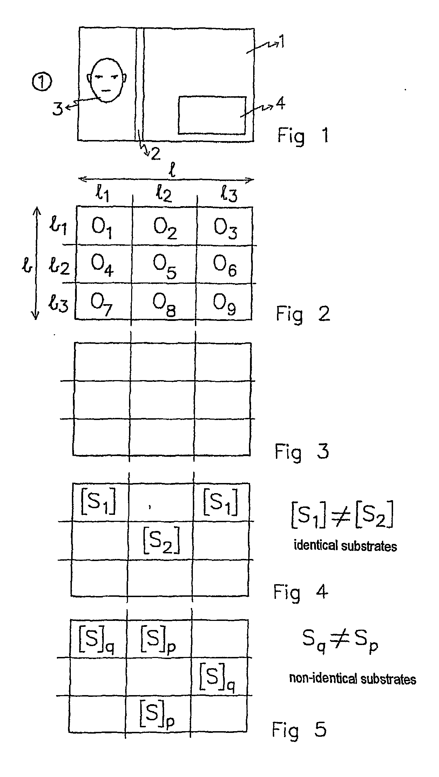

Figure 1 shows a security document 1, in this case a bank note. The security document is provided with security features known per se, such as a security thread 2 and a watermark 3. An embodiment of a security feature 4 according to the invention is applied locally, and in the example given here covers only a limited part of the document 1. The feature 4 can be completely uniform and continuous, but it is also possible to introduce discontinuities within the feature. The following figures give a number of illustrative examples thereof. In these figures and examples, for the sake of clarity and simplicity, only one or two enzymatic conversions take place. The number of examples however can be easily increased by the person skilled in the art by for example including different enzyme systems within the feature and/or by taking enzymes with the same specificity but which are of different origins, as a result of which the catalytic parameters may differ under the given conditions.

The examples are intended to illustrate the invention, viz. at least one particular enzyme reaction with at least one predetermined conversion with a detectable change, in particular after the addition of a starting reagent under known conditions with regard to the final composition and all relevant factors which may influence an enzymatic reaction, with the enzyme having good resistance to the normal user conditions. Not normal user condition, like an attempt to tamper, may also be detected by the system as the expected enzymatic conversion will partially or fully fail then.

In the examples given the starting reagent contains at least one enzyme. The examples can be easily increased, however, by the addition of variants in which the security document is provided with at least the enzyme, and in which the starting reagent does not contain the enzyme.

Figure 2 shows a general embodiment of the security feature 4. Each of the given component regions $O_1$ to $O_9$ inclusive may have an identical feature composition; if all the component regions have an identical composition, then the feature is uniform. The component regions may also differ from each other in composition, however; in that case there are one or more discontinuities in the feature.

Any possible combination of $l_1$, $l_2$, $l_3$ and $b_1$, $b_2$, $b_3$ is possible in this example within the boundary conditions: $l_1 + l_2 + l_3 = l$ and $b_1 + b_2 + b_3 = b$, for the rectangular arrangement of the feature that is given here, where 1 stands for length and b stands for width. It is obvious that the feature can be divided into more parts than the nine regions given here (generally in such a subdivision: 1 can be divided into $l_1$ to $l_x$ inclusive, with the length $0 \leq l_n \leq l$ and $\Sigma l_n = 1$; b can be divided into $b_1$ to by inclusive, with the width $0 \leq b_n \leq b$ and $\Sigma b_n = b$; the number of component regions $\leq x*y$ in a division of this kind. The given shape of the feature is only used because it represents a simple geometric example; however, any shape is conceivable with any subdivision.

In a particular embodiment (Figure 3) the emphasis is placed on possible variations in the composition on/in the different component surfaces. Variation may lie in one or more factors that have an influence on the rate of an enzymatic conversion like: the substrate concentration and/or the kind of substrate and/or an inhibitor concentration and/or the kind of inhibitor and/or a co-factor concentration and/or a co-enzyme concentration and/or an activator concentration and/or the pH and/or the ionic strength and/or the ionic composition, or specific combinations of all these factors. The concentration of the enzyme is here disregarded and is assumed to be constant in these examples only for the sake of convenience. A person skilled in the art can easily extend the number of possibilities/combinations further.

It should be noted here that the total concentrations of factors may sometimes be identical but that the effective concentrations thereof may nevertheless be different. Thus one can use a particular fixed concentration of a co-factor, e.g. in the form of a metal ion, but at the same time introduce variations in the presence/concentrations of a metal complexing agent which can form a complex with the relevant metal ion (e.g. EDTA). The free metal ion concentration is usually determinative for an enzymatic reaction rate; this rate will then also be dependent on the EDTA concentration at a given total concentration of the metal. Some of these free metal ions may be complexed with one of the reactants and/or with the enzyme; these ions can thus not really be called free but they are effective in the enzyme-catalysed reaction, unlike the EDTA- complexed ions. A known example of enzymatic reactions which are dependent on a metal are those in which at least one phosphate group is involved. These reactions are often $Mg^{2+}$ ion dependent ( $Mg^{2+}$ complexes with one or more negative charges of phosphate groups of the compounds which are involved in the reaction). Although the total concentrations of, for example, the $Mg^{2+}$ ion may be identical, one obtains different conversion rates in the presence of a fixed $Mg^{2+}$ concentration in the presence of varying EDTA concentrations.

A particular embodiment is given in Fig. 4, where the substrate concentration for at least one kind of enzymatic conversion in at least one of the component regions differs from those in the other regions, with the other conditions/ concentrations remaining constant. As a result of this, at least one deviant enzymatic conversion rate and/or final state occurs during the testing in at least one of the different component regions. It is also possible to complex a metal ion that is part of the active enzyme. The enzyme then will be fully or partially inactive; by adding an excess of the metal ion in the last addition the activity might be restored. This again is an example of the numerous possibilities to create a very complicated test system, which hardly can be counterfeited.

Another particular embodiment is given in Fig. 5, where in at least one of the component regions a different substrate is used for a particular enzymatic conversion, as a result of which this region exhibits a different signal after testing. For example one can use substrate $S_P$ in at least component regions $O_2$ and $O_8$ (these component regions are chosen fully arbitrarily), while at least one of the other component surfaces (e.g. $O_1$ and $O_6$) comprise another substrate $S_q$. The visible and/or measurable signal in the regions $O_2$ and $O_8$ may differ from that in the regions $O_1$ and $O_6$ with $S_q$ because of a different conversion rate and/or different spectral properties of the substrates and/or respective products; most likely the kinetic parameters will be different.

It is for example possible to cause one conversion to be associated with a measurable and/or directly visually perceptible colour change, while the other conversion is associated with a measurable and/or perceptible change for which for instance uv light is required to be seen/measured; this signal might also be a fluorescent one.

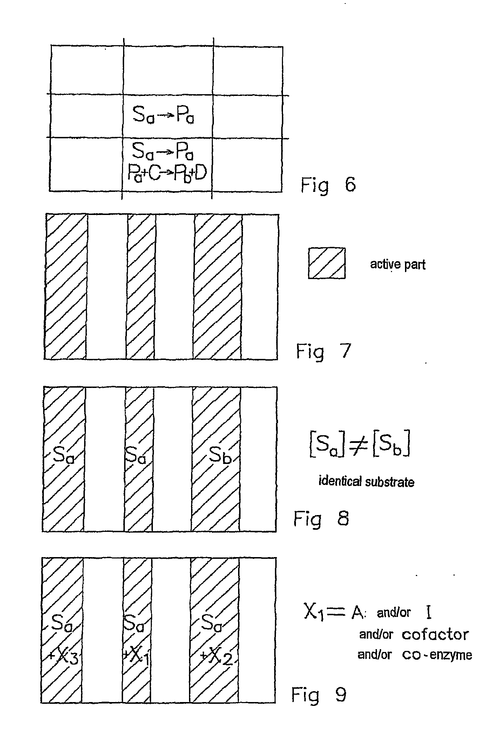

In another particular embodiment according to Fig. 6, one uses a linked reaction in at least one component region, using a particular substrate, and in another component region the same substrate is used without a linked reaction, so that another signal is generated locally. Thus, for example, in component region $O_5$ (arbitrary example) one can obtain the visible and/or measurable conversion of $S_a \text{----} > P_a$ and in component region $O_8$ (arbitrary example) the visible and/or measurable conversion of $S_a \text{----} > P_a$ followed by $P_a + C \text{----} > P_b + D$ or $P_a \text{----} > P_b$, with a visible and/or measurable change occurring in the second reaction; the different signals may differ in colour and/or intensity and/or emission. Here again the conversions can be both visually perceptible and/or measurable via a (spectro- )photometer and/or via a fluoro (spectro)photometer measuring in the uv and/or visible region and/or (near) infra red. In the example given only one follow-up reaction is indicated, but use can also be made of several follow-up reactions within a component region, or of follow-up reactions that differ from each other in different component regions.

As indicated earlier via follow-up reactions one can sometimes regenerate one of the reactants (substrates and/or co-enzymes), so that a relatively low initial concentration of this reactant will suffice. In addition, by taking products away from the first reaction(s), the degree of substrate conversion of this/these first reaction(s) will be favourably affected. Additional enzymes may be required for follow-up reactions.

A particular embodiment is given in Fig. 7, where the feature is characterized by a discontinuous distribution of the factors/reactants present in the feature and needed for the enzymatic conversion, in the form of an (in)visible and/or measurable bar-code. In each of the bars in which the a reaction might take place one can make the factors/ reactants composition identical but one can also make them differ. If the composition within the active parts of the feature is identical, then after the reaction occurs one will for example see a bar-code with bars with the same perceptible and/or measurable colour intensity; it may also be that all the initially perceptible/measurable bars equally disappear at the place where the reaction takes place. This is all entirely determined by the composition of the feature. The final intensity of the bars may also differ, depending on the original composition or by the last addition(s).

A particular embodiment is given in Fig. 8, in which the substrate concentration of at least one of the bars is different from that in the others (the composition and conditions being otherwise identical). This results in at least one different signal change in this bar compared with those in the other bars; this difference may lie in the rate at which a signal change occurs and/or in the final degree of the signal change.

A particular embodiment is given in Fig. 9 by introducing differences in composition between the bars, which locally alter the enzymatic conversion rate, such as the presence/absence of an inhibitor and/or a co-factor and/or a co-enzyme and/or an activator. The concentrations of all these factors, if present, can also be varied for each bar. At the same time, apart from these variations, it is also possible to vary the ionic strength and the ionic composition locally.

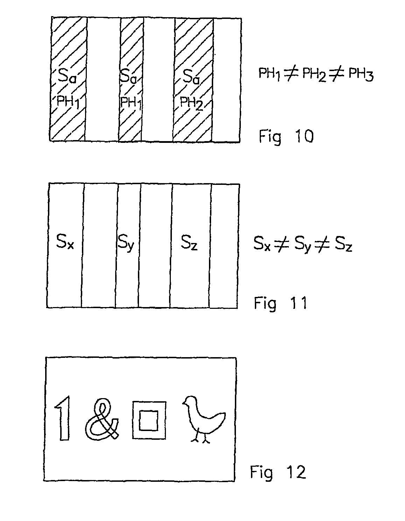

In a particular embodiment (Fig. 10) the enzymatic reactions in the various component regions are carried out under different reaction conditions, more specifically different pH values, so that the conversion rate in the component regions differs, depending among other things on the enzymes and substrates used.

In a particular embodiment (Fig. 11) the type of substrate in each bar is different, so that a wide variety of changes can occur in the total feature. The substrates might all be convertible by one type of enzyme, but at different rates and/or with different signal changes, or the substrates may be (almost) identical (e.g. in as far as the gross formula is concerned) but have to be converted by different enzymes.

Figure 12 shows an embodiment in which the feature is made up of specific forms, such as signs and/or symbols and/or figures. All the forms or parts within the feature may have identical properties, in so far as enzymatic conversions are concerned, but equally well at least one form or at least on sub-part may be different from the others, and in the extreme case all the forms are different from each other in so far as the rate and/or type of enzymatic conversion is concerned.

The addition of any starting reagent/composition can be carried out via metering systems that are known for this purpose. One can also apply a starting reagent with a pen; a stamping pad, or by inkjet printing, for example. Simply drawing a line with a pen or application of a stamp can suffice for a qualitative test.

When in the specification including the claims the word 'concentration' is used it may be understood as a certain amount per unit of surface area and/or per unit of volume.

**Claims**

1. Method for authenticating a security document, where the security document comprises at least one of an enzyme and a substrate suitable for the enzyme, which method comprises the steps of

   a) carrying out an enzymatic conversion between the enzyme and the substrate on the security document,
   b) assessing a detectable change resulting from the enzymatic conversion,
   wherein the genetic information for the enzyme is derived from an extremophilic micro-organism.

2. Authentication system for authenticating security documents, comprising at least an enzyme and a substrate suitable for the enzyme, so that the enzyme is capable of an enzymatic conversion in which a detectable resultant change occurs, and the security document comprises at least one of the enzyme and the substrate, wherein the genetic information for the enzyme is derived from an extremophilic micro-organism.

3. Security document, such as a deed, bank note, value document, tickets, voucher, fiscal stamp, identity document, visa, bank card, label and packaging in particular for pharmaceutical, cosmetic, electronic and food industry, provided with a security feature based on an enzymatic conversion between an enzyme and a substrate, wherein the security feature comprises an enzyme the genetic information of which is derived from an extremophilic micro-organism.

4. Method according to claim 1, or authentication system according to claim 2, or security document according to claim 3, wherein the genetic information for the enzyme is derived from a hyperthermophilic or thermophilic micro-organism.

5. Method according to claim 1 or 4, or authentication system according to claim 2 or 4, or security document according to claim 3 or 4, wherein the security document is provided with the enzyme, preferably in a coating.

6. Method according to any of the claims 1, 4, 5, or authentication system according to any of the claims 2, 4, 5 or security document according to any of the claims 3-5, wherein the enzyme is stabilized.

7. Method according to any of the claims 1, 4-6, or authentication system according to any of the claims 2, 4-6, or security document according to any of the claims 3-6, wherein the enzyme is present in a starting reagent, which reagent preferably comprises a substance and/or a means for stabilizing the enzyme.

8. Method according to any of the claims 1, 4-7, or authentication system according to any of the claims 2, 4-7, or security document according to any of the claims 3-7, wherein the enzyme is bound to a solid support or incorporated in a solid particle.

9. Method according to any of the claims 1, 4-8, or authentication system according to any of the claims 2, 4-8, or security document according to any of the claims 3-8, wherein a product of the enzymatic conversion is used as a reagent in a chemical and/or enzymatic follow-up reaction.

10. Method according to any of the claims 1, 4-9, or authentication document according to any of the claims 2, 4-9, or security document according to any of the claims 3-9, wherein at least two different enzymes are provided in regions of the surface of the security document at least partially overlapping each other.

**Patentansprüche**

1. Verfahren zur Authentifizierung eines Sicherheitsdokuments, wobei das Sicherheitsdokument mindestens eines aus einem Enzym und einem für das Enzym geeigneten Substrat umfasst, wobei das Verfahren die Schritte umfasst

   a) Durchführen einer enzymatischen Umwandlung zwischen dem Enzym und dem Substrat auf dem Sicherheitsdokument,
   b) Bewerten einer nachweisbaren Veränderung, welche aus der enzymatischen Umwandlung resultiert,
   wobei die Erbinformation für das Enzym von einem extremophilen Mikroorganismus abstammt.

2. Authentif'izierungssystem zur Authentifizierung von Sicherheitsdokumenten, umfassend mindestens ein Enzym und ein für das Enzym geeignetes Substrat, so dass das Enzym für eine enzymatische Umwandlung geeignet ist, in welcher eine nachweisbare resultierende Veränderung stattfindet, und wobei das Sicherheitsdokument mindestens eines aus dem Enzym und dem Substrat umfasst, wobei die Erbinformation für das Enzym von einem extremophilen Mikroorganismus abstammt.

3. Sicherheitsdokument, wie etwa eine Urkunde, ein Geldschein, Wertdokument, Tickets, Beleg, Fiscalmarke, Identitätsdokument, Visum, Bankkarte, Etikett und Verpackung, insbesondere für die Pharma-, Kosmetik-, Elektronik- und Nahrungsmittelindustrie, ausgestattet mit einem Sicherheitsmerkmal auf der Basis einer enzymatischen Umwandlung zwischen einem Enzym und einem Substrat, wobei das Sicherheitsmerkmal ein Enzym umfasst, dessen Erbinformation von einem extremophilen Mikroorganismus abstammt.

4. Verfahren gemäß Anspruch 1 oder Authentifizierungssystem gemäß Anspruch 2 oder Sicherheitsdokument gemäß Anspruch 3, wobei die Erbinformation für das Enzym von einem hyperthermophilen oder einem thermophilen Mikroorganismus abstammt.

5. Verfahren gemäß Anspruch 1 oder 4 oder Authentifizierungssystem gemäß Anspruch 2 oder 4 oder Sicherheitsdokument gemäß Anspruch 3 oder 4, wobei das Sicherheitsdokument mit dem Enzym ausgestattet ist, vorzugsweise in einer Beschichtung.

6. Verfahren gemäß einem der Ansprüche 1, 4, 5 oder Authentifizierungssystem gemäß einem der Ansprüche 2, 4, 5 oder Sicherheitsdokument gemäß einem der Ansprüche 3-5, wobei das Enzym stabilisiert ist.

7. Verfahren gemäß einem der Ansprüche 1, 4-6 oder Authentifizierungssystem gemäß einem der Ansprüche 2, 4-6 oder Sicherheitsdokument gemäß einem der Ansprüche 3-6, wobei das Enzym in einem Ausgangsreagens vorliegt, wobei das Reagens vorzugsweise eine Substanz und/oder ein Mittel zum Stabilisieren des Enzyms umfasst.

8. Verfahren gemäß einem der Ansprüche 1, 4-7 oder Authentif'izierungssystem gemäß einem der Ansprüche 2, 4-7 oder Sicherheitsdokument gemäß einem der Ansprüche 3-7, wobei das Enzym an einen festen Träger gebunden oder in ein festes Teilchen eingebettet ist.

9. Verfahren gemäß einem der Ansprüche 1, 4-8 oder Authentifizierungssystem gemäß einem der Ansprüche 2, 4-8 oder Sicherheitsdokument gemäß einem der Ansprüche 3-8, wobei ein Produkt der enzymatischen Umwandlung als ein Reagens in einer chemischen und/oder enzymatischen Folgereaktion verwendet wird.

10. Verfahren gemäß einem der Ansprüche 1, 4-9 oder Authentifizierungssystem gemäß einem der Ansprüche 2, 4-9 oder Sicherheitsdokument gemäß einem der Ansprüche 3-9, wobei mindestens zwei verschiedene Enzyme in Bereichen der Oberfläche des Sicherheitsdokuments bereitgestellt sind, die mindestens teilweise miteinander überlappen.

**Revendications**

1. Procédé d'authentification d'un document de sécurité, où le document de sécurité comprend au moins un élément parmi une enzyme et un substrat approprié pour l'enzyme, lequel procédé comprend les étapes consistant à

   a) réaliser une conversion enzymatique entre l'enzyme et le substrat sur le document de sécurité,
   b) estimer un changement détectable résultant de la conversion enzymatique,
   où les informations génétiques pour l'enzyme sont dérivées d'un micro-organisme extrémophile.

2. Système d'authentification permettant d'authentifier des documents de sécurité, comprenant au moins une enzyme et un substrat approprié pour l'enzyme, de sorte que l'enzyme est capable d'une conversion enzymatique dans laquelle un changement résultant détectable se produit, et le document de sécurité comprend au moins un élément parmi l'enzyme et le substrat, où les informations génétiques pour l'enzyme sont dérivées d'un micro-organisme extrémophile.

3. Document de sécurité, tel qu'un acte notarié, un billet de banque, un document de valeur, des tickets, un bon, un timbre fiscal, un document d'identité, un visa, une carte bancaire, une étiquette et un emballage en particulier pour les industries pharmaceutiques, cosmétiques, électroniques et alimentaires, pourvu d'une particularité de sécurité basée sur une conversion enzymatique d'une enzyme et d'un substrat, où la particularité de sécurité comprend une enzyme dont les informations génétiques sont dérivées d'un micro-organisme extrémophile.

**4.** Procédé selon la revendication 1, ou système d'authentification selon la revendication 2, ou document de sécurité selon la revendication 3, dans lequel les informations génétiques pour l'enzyme sont dérivées d'un micro-organisme hyperthermophile ou thermophile.

**5.** Procédé selon la revendication 1 ou 4, ou système d'authentification selon la revendication 2 ou 4, ou document de sécurité selon la revendication 3 ou 4, dans lequel le document de sécurité est pourvu de l'enzyme, de préférence dans un revêtement.

**6.** Procédé selon l'une quelconque des revendications 1, 4, 5, ou système d'authentification selon l'une quelconque des revendications 2, 4, 5, ou document de sécurité selon l'une quelconque des revendications 3 à 5, dans lequel l'enzyme est stabilisée.

**7.** Procédé selon l'une quelconque des revendications 1, 4 à 6, ou système d'authentification selon l'une quelconque des revendications 2, 4 à 6, ou document de sécurité selon l'une quelconque des revendications 3 à 6, dans lequel l'enzyme est présente dans un réactif de départ, lequel réactif comprend de préférence une substance et/ou un moyen permettant de stabiliser l'enzyme.

**8.** Procédé selon l'une quelconque des revendications 1, 4 à 7, ou système d'authentification selon l'une quelconque des revendications 2, 4 à 7, ou document de sécurité selon l'une quelconque des revendications 3 à 7, dans lequel l'enzyme est liée à un support solide ou incorporée dans une particule solide.

**9.** Procédé selon l'une quelconque des revendications 1, 4 à 8, ou système d'authentification selon l'une quelconque des revendications 2, 4 à 8, ou document de sécurité selon l'une quelconque des revendications 3 à 8, dans lequel le produit de la conversion enzymatique est utilisé comme réactif dans une réaction de suivi chimique et/ou enzymatique.

**10.** Procédé selon l'une quelconque des revendications 1, 4 à 9, ou système d'authentification selon l'une quelconque des revendications 2, 4 à 9, ou document de sécurité selon l'une quelconque des revendications 3 à 9, dans lequel au moins deux enzymes différentes sont disposées dans des régions de la surface du document de sécurité se chevauchant au moins partiellement les unes les autres.

Fig 1

Fig 2

Fig 3

$[S_1] \neq [S_2]$

identical substrates

Fig 4

$S_q \neq S_p$

non-identical substrates

Fig 5

$$S_a \rightarrow P_a$$

$$S_a \rightarrow P_a$$
$$P_a + C \rightarrow P_b + D$$

Fig 6

active part

Fig 7

$$[S_a] \neq [S_b]$$

identical substrate

$S_a$   $S_a$   $S_b$

Fig 8

$$X_1 = A \text{ and/or } I$$
and/or cofactor
and/or co-enzyme

$S_a$   $S_a$   $S_a$
$+X_3$   $+X_1$   $+X_2$

Fig 9

EP 1 839 279 B1

$PH_1 \neq PH_2 \neq PH_3$

Fig 10

$S_x \neq S_y \neq S_z$

Fig 11

Fig 12

17

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 9014441 A
- EP 0327163 A
- US 5139812 A
- US 5429952 A
- US 5643728 A
- US 5942444 A

- DE 10205506
- WO 03038000 A
- WO 8706383 A
- US 6083696 A
- US 6287765 B

**Non-patent literature cited in the description**

- **Koepsel ; Russel.** *Science,* vol. 309, 377, www.sciencemag.org

- **Alan Fersht.** Structure and mechanism in protein science. W. H. Freeman and Company, 1999, 362